# EUROPÄISCHE PATENTSCHRIFT

(11) **EP 2 534 116 B1**
(45) Veröffentlichungstag und Bekanntmachung des Hinweises auf die Patenterteilung: **24.09.2014**
(21) Anmeldenummer: 11701848.1
(22) Anmeldetag: 07.02.2011
(51) Int. Cl.: C07C 5/27, C07B 37/08

(54) **VERFAHREN ZUR HERSTELLUNG M-SUBSTITUIERTER ALKYLTOLUOLE DURCH ISOMERISIERUNG MIT IONISCHEN FLÜSSIGKEITEN ALS KATALYSATOREN**
METHOD FOR PRODUCING AN M-SUBSTITUTED ALKYL TOLUENE BY ISOMERIZIATION USING IONIC LIQUIDS AS CATALYSTS
PROCÉDÉ DE PRÉPARATION D'ALKYLTOLUÈNES M-SUBSTITUÉS PAR ISOMÉRISATION AVEC DES LIQUIDES IONIQUES EN TANT QUE CATALYSEURS

(30) Priorität: 11.02.2010 EP 10153317
(43) Veröffentlichungstag der Anmeldung: 19.12.2012
(73) Patentinhaber: BASF SE, 67056 Ludwigshafen (DE)
(72) Erfinder: LANVER, Andreas, 68165 Mannheim (DE); EBEL, Klaus, 68623 Lampertheim (DE); BECK, Karl, 76684 Östringen (DE); PELZER, Ralf, 37699 Fürstenberg (DE); BOTZEM, Jörg, 67117 Limburgerhof (DE); GRIESBACH, Ulrich, 68305 Mannheim (DE)
(86) Internationale Anmeldenummer: PCT/EP2011/051732
(87) Internationale Veröffentlichungsnummer: WO 2011/098418

(56) Entgegenhaltungen:
- JP-B2- 2 738 093
- GEORGE A. OLAH ET AL.,: "BORON, ALUMINUM, AND GALLIUM TRIS(TRIFLUOROMETHANESULFONATE) (TRIFLATE): EFFECTIVE NEW FRIEDEL-CRAFTS CATALYSTS", J. AM.CHEM.SOC., Bd. 110, Nr. 8, 1. Januar 1988 (1988-01-01), Seiten 2560-2565, XP001033717, in der Anmeldung erwähnt
- PETER KOVACIC, ET AL.,: "AMINATION OF m-DIALKYLBENZENES WITH TRICHLORAMINE-ALUMINIUM CHLORIDE", JOURNAL OF ORGANIC CHEMISTRY, Bd. 32, 1967, Seiten 585-588, XP002650388,
- NESTEROVA T ET AL: "Contributions of Enthalpy and Entropy Factors to Isomerization Equilibrium of Isopropyl- and Cyclohexylbenzenes", RUSSIAN JOURNAL OF APPLIED CHEMISTRY, Bd. 72, Nr. 11, 1. Januar 1999 (1999-01-01), Seiten 1884-1890, XP009149500, ISSN: 1070-4272

## Beschreibung

Gegenstand der vorliegenden Erfindung ist ein Verfahren zur Herstellung m-substituierter Alkyltoluole durch Isomerisierung von p-substituierten Alkyltoluolen. Substituierte Alkyltoluole sind wichtige Zwischenprodukte bei der Synthese von Riechstoffen vom Typus 2-Methyl-3-Phenylpropanal, wie z.B. Lysmeral ® (BASF SE).

Die in der vorliegenden Erfindung verwendeten ionischen Flüssigkeiten enthaltend vorzugsweise ein Ammonium- oder Imidazoliumsalz und ein Metallhalogenid, sind in der Literatur beschrieben und werden u.a. in Friedel-Crafts-Alkylierungen eingesetzt (Übersicht: P. Wasserscheid, T. Welton, lonic Liquids in Synthesis, Wiley-VCH, 2003, S. 174ff).

In der JP 2738093 wird die Isomerisierung von 4-tert-Butyltoluol zu 3-tert-Butyltoluol mit AlCl₃ als Katalysator beschrieben. Gegenüber 4-tert-Butyltoluol ist 3-tert-Butyltoluol das thermodynamisch günstigere Produkt. Mit AlCl₃ als Katalysator bildet sich ein m/p-Isomerengemisch im Gleichgewicht mit einem m/p-Verhältnis von ca. 2/1 nach einer Reaktionszeit von 1 h bei Raumtemperatur. Neben diesem Gleichgewicht existiert ein weiteres konkurrierendes Gleichgewicht, welches zur Bildung von Toluol und 3,5-Di-tert-butyltoluol führt. Um die Menge an 3,5-Di-tert-butyltoluol zu reduzieren wurde in der JP 2738093 die Zugabe von Toluol (1 Gewichtsäquivalent) beschrieben.

Olah et al. haben die Isomerisierung von 4-tert-Butyltoluol zu 3-tert-Butyltoluol bzw. die Alkylierung von Toluol mit tert-Butylchlorid beschrieben (J. Am.Chem.Soc. 1988, 110, 2560-2565). Dabei wurden als Katalysatoren Tris(trifluormethansulfonate) (Triflate) von Bor, Aluminium und Gallium [B(OTf)₃, Ga(OTf)₃ und Al(OTf)₃] eingesetzt. Es stellte sich auch hier ein Gleichgewicht von 3-tert-Butyltoluol / 4-tert-Butyltoluol von ca. 2 / 1 ein.

Bei der technischen Umsetzung dieser Verfahren ist von Nachteil, dass man den Katalysator durch eine aufwändige Fest-Flüssig-Trennoperation abtrennen muss oder diesen durch eine wässrige Aufarbeitung hydrolysieren muss. Dabei fällt eine signifikante Menge an chloridhaltigem Abfall und an Metallsalzen an.

Demgegenüber ermöglicht das erfindungsgemäße Verfahren, dass man m-substituierte Alkyltoluole durch Isomerisierung von p-substituierten Alkyltoluolen in Gegenwart von ionischen Flüssigkeiten effizient herstellen kann. Vorteile der erfindungsgemässen Verfahrensweise sind:
1) die Abtrennung des Katalysators gelingt durch eine technisch weniger aufwändige Flüssig-Flüssig-Trennoperation;
2) die einfache Rückführbarkeit des Katalysators (da keine wässrige Aufarbeitung notwendig ist, die den Katalysator im Falle von festem AlCl₃ zerstört);

Die vorliegende Erfindung betrifft ein Verfahren zur Herstellung von in m-Stellung substituierten Alkyltoluolen, welche durch Isomerisierung von in p-Stellung substituierten Alkyltoluolen erhalten werden können. Die Isomerisierung erfolgt an ionischen Flüssigkeiten als Katalysatoren. Gegenstand der Erfindung ist somit ein Verfahren zur Herstellung m-substituierter Alkyltoluolen der Formel (I) 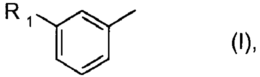 worin R₁ C₁-C₅-Alkyl bedeutet, welches dadurch gekennzeichnet ist, dass man ein p-substituiertes Alkyltoluol der Formel (II) 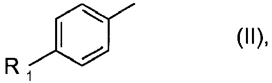 worin R₁ die unter Formel (I) angegebene Bedeutung hat in Gegenwart von ionischen Flüssigkeiten zu einem m-substituierten Alkyltoluol der Formel (I) isomerisiert.

Es wurde überraschend gefunden, dass ionische Flüssigkeiten die Isomerisierung katalysieren. Besonders geeignete aktive ionische Flüssigkeiten sind z.B. Mischungen enthaltend die Komponenten (a) und (b), wobei
(a) eine erste Komponente der Formel MX₃ oder MX₂ ist, worin M Metall, wie z.B. Al**,** In, Zn, Fe, und X Halogen, wie z.B. Chlor, Brom und Jod, bedeuten und,
(b) eine zweite Komponente ist, ausgewählt aus der folgenden Gruppe: Alkylammoniumhalogenid, Imidazoliumhalogenid, Pyridiniumhalogenid, Phosphoniumhalogenid und Mischungen dieser Verbindungen.

Bevorzugt sind ionische Flüssigkeiten bestehend aus der Mischung der Komponenten (a) und (b).

Das Verhältnis der beiden Komponenten (a)/(b) ist > 1/1.

Der Term "ionische Flüssigkeit" bezeichnet Mischungen der Verbindungen (a) und (b) wie oben beschrieben, welche nachdem sie homogenisiert und ggf. aufgeschmolzen sowie wieder abgekühlt wurden bei einer Temperatur von <100°C flüssig sind.

Bevorzugte Komponenten (a) sind AlCl₃, AlBr₃, ZnCl₂, FeBr₃ und FeCl₃. Die Komponente (a) ist besonders bevorzugt AlCl₃. Die Komponente (b) ist bevorzugt ein Imidazoliumhalogenid, wie z.B. durch C₁-C₁₀-Alkyl ein oder mehrfach substituiertes Imidazoliumhalogenid, oder ein Alkylammoniumhalogenid, wie z.B. durch C₁-C₆-Alkyl di-, tri-oder tetra substituiertes Alkylammoniumhalogenid. Spezifische Beispiele für besonders bevorzugte Verbindungen (b) sind: 1-Ethyl-3-methylimidazoliumchlorid, 1-Butyl-3-methylimidazoliumchlorid, 1-Hexyl-3-methylimidazoliumchlorid, 1-Methylimidazolium-chlorid, Triethylammoniumchlorid, Trimethylammoniumchlorid, Tetramethylammoni-umchlorid, Tetraethylammoniumchlorid, 1-Butylpyridiniumchlorid und 1-Methylpyridiniumchlorid.

Das Verhältnis der Komponenten (a) / (b) ist > 1 / 1. Bevorzugt ist ein Verhältnis von > 1/1 und < 3 / 1. Besonders bevorzugt ist ein Verhältnis von > 1,5/ 1 und <2,1 / 1. Ganz besonders bevorzugt ist ein Verhältnis von 2 / 1.

Die Herstellung des Katalysators erfolgt typischerweise dadurch, dass Komponente (a) oder Komponente (b) in einem Lösungsmittel vorgelegt wird und dann Komponente (b) oder Komponente (a) zudosiert wird. Nach einer Reaktionszeit von 1 -12 Stunden bei einer Temperatur zwischen 20 -100°C bildet sich die ionische Flüssigkeit. Die ionische Flüssigkeit kann entweder durch Phasentrennung von der Lösungsmittelphase abgetrennt werden oder komplett als homogene Lösung des Katalysators im Lösungsmittel in der Isomerisierung eingesetzt werden. Bevorzugte Lösungsmittel sind aromatische Kohlenwasserstoffe oder chlorierte Lösungsmittel. Besonders bevorzugt sind Benzol, Toluol, Xylole, Mesitylen, 4-tert-Butyltoluol und Dichlormethan. Ganz besonders bevorzugt ist Toluol.

Die ionische Flüssigkeit als Katalysator wird in der Isomerisierung in einer Menge von 0,1 - 100 mol%, bezogen auf das Ausgangsmaterial der Formel (II), eingesetzt. Die bevorzugte Katalysatormenge ist 0,1 - 10mol%. Besonders bevorzugt sind 0,5 - 5 mol%.

Die Isomerisierung erfolgt bei Temperaturen zwischen 0°C und 100°C. Besonders bevorzugt sind Temperaturen zwischen 10°C und 50°C. Die Reaktionszeiten betragen 1 Minute bis 10 Stunden. Besonders bevorzugt sind Reaktionszeiten zwischen 30 Minuten und 3 Stunden.

Die Isomerisierung kann lösungsmittelfrei oder in einem Lösungsmittel durchgeführt werden. Geeignete Lösungsmittel sind: Toluol, Xylole, Dichlormethan, Chlorbenzol, Hexan, Heptan. Besonders bevorzugt ist Toluol.

In dem erfindungsgemäßen Verfahren wird als Ausgangsstoff ein p-substituiertes Al-kyltoluol der Formel (II)eingesetzt. Der Alkylrest R₁ ist ein C₁-C₅-Alkylrest, wie z.B. ein Methyl-, Ethyl-, Isopropyl-, Isobutyl-, tert-Butyl- oder Isopentyl-Rest.

Eine bevorzugte Ausführungsform des erfindungsgemäßen Verfahrens, ist dadurch gekennzeichnet, dass der Rest R₁ in der Formel (II)bevorzugt C₁-C₄-Alkyl, besonders bevorzugt Ethyl, Isopropyl, oder tert-Butyl bedeutet.

Bevorzugte Ausgangsstoffe für die Isomerisierung sind die folgenden Substrate: 4-tert-Butyltoluol, 4-Isopropyltoluol, 4-Ethyltoluol. Daraus ergeben sich die folgenden m-Isomere als Hauptprodukte (Isomerenverhältnis m / p > 1 / 1) der Reaktion: 3-tert-Butyltoluol, 3-Isopropyltoluol, 3-Ethyltoluol. Besonders bevorzugt ist das Substrat 4-tert-Butyltoluol.

Die Reaktion wird in der Regel so durchgeführt, dass ein Gemisch bestehend aus Toluol, p-alkylsubstituiertem Toluol und di-alkylsubstituiertem Toluol mit der ionischen Flüssigkeit umgesetzt wird. Nach beendeter Reaktion erfolgt eine Trennung der Phasen. Die Katalysatorphase kann in der nächsten Charge wieder eingesetzt werden. Die zweite Phase enthält das Wertprodukt (m-substituiertes Alkyltoluol), sowie Toluol, das p-substituierte Alkyltoluol und ein dialkylsubstituiertes Alkyltoluol. Zur weiteren Aufarbeitung wird diese Phase einer wässrigen Wäsche gefolgt von einer Destillation oder direkt einer Destillation unterworfen. Die Isolierung der m-substituierten Alkyltoluole erfolgt in der Regel durch Destillation. Die bei der Reaktion entstehenden Komponenten Toluol und Dialkyltoluole sind Nebenprodukte, die destillativ abgetrennt und der Isomerisierungsreaktion wieder zugeführt werden können. Durch die Rückführung dieser Produkte in die Reaktionsmasse wird das Gleichgewicht zwischen dem m-substituierten Alkyltoluol und dem p-substituierten Alkyltoluol immer wieder neu eingestellt, wodurch ein erhöhter Anteil an dem gewünschten m-substituierten Produkt erhalten wird.

Ein weiterer Gegenstand der Erfindung ist die Herstellung der aus den m-substituierten Alkyltoluolen der Formel (I) durch eine Reaktionssequenz bestehend aus Oxidation, Aldolkondensation und Hydrierung zugänglichen Wertprodukte der Formel (V) 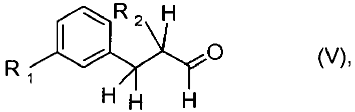 worin R₁ die unter Formel (I) angegebene Bedeutung hat und R₂ Wasserstoff oder C₁-C₅-Alkyl, vorzugsweise Wasserstoff oder Methyl, ist, die als Duftstoffe und Aromachemikalien relevant sind.

Der Alkylrest R₂ ist ein C₁-C₅-Alkylrest, wie z.B. ein Methyl-, Ethyl-, Isopropyl-, n-Propyl, Isobutyl-, tert-Butyl-, n-Butyl-, n-Pentyl- oder Isopentyl-Rest. Vorzugsweise ist der Rest R₂ Wasserstoff oder Methyl.

Aus den erfindungsgemäß erhältlichen m-substituierten Alkyltoluolen der Formel (I) 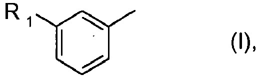 worin R₁ C₁-C₅-Alkyl, vorzugsweise Ethyl, Isopropyl, oder tert-Butyl bedeutet, kann durch Oxidation nach an sich bekannten Verfahren (z.B. elektrochemische Oxidation von substituierten Toluolen zu substituierten Benzaldehyden gemäss DE2948455) der m-alkylsubstituierte Benzaldehyd der Formel (III) 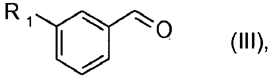 worin R₁ die unter Formel (I) angegebene Bedeutung hat, gebildet werden. Eine nachfolgende Aldolkondensation von substituierten Benzaldehyden der Formel (III) mit z.B. einem Aldehyd der Formel (IIIa) 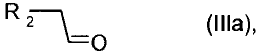 worin R₂ Wasserstoff oder C₁-C₅-Alkyl bedeutet, zu substituierten Zimtaldehyden der Formel (IV) ist z.B. in DE19949672 beschrieben 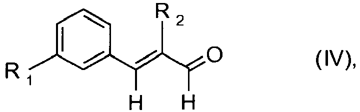 worin R₁ die unter Formel (III) angegebene Bedeutung hat und R₂ Wasserstoff oder C₁-C₅-Alkyl, vorzugsweise Wasserstoff oder Methyl bedeutet. Die Hydrierung von substituierten Zimtaldehyden der Formel (IV) zu substituierten Phenylpropanalen der Formel (V) ist z.B. in DE102005049568 beschrieben 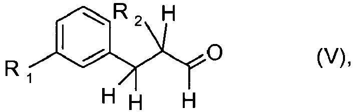 worin R₁ und R₂ die unter Formel (IV) angegebenen Bedeutungen haben.

Die gemäß dem Verfahren erhaltenen Aldehyde der Formel (V) sind zum Teil bekannt und teilweise neue Duft- und Aromastoffe.

In den Verbindungen vom Typ (III), (IV) und (V) ist R₁ in m-Stellung an den Phenylring gebunden und bedeutet C₁-C₅-Alkyl, vorzugsweise C₁-C₄-Alkyl, besonders bevorzugt Ethyl, Isopropyl und tert-Butyl, und R₂ bedeutet Wasserstoff oder C₁-C₅-Alkyl, vorzugsweise Wasserstoff oder Methyl. Ganz besonders bevorzugt bedeutet R₁ tert-Butyl.

Die Erfindung wird durch die folgenden Beispiele näher veranschaulicht ohne sie darauf zu beschränken. In den Beispielen verstehen sich alle Angaben in % als Gew.-%. (Abkürzungen: BMIM = 1,3-Butylmethylimidazolium; EMIM = 1,3-Ethylmethylimidazolium; HMIM = 1-Methylimidazolium; TEA = Triethylammonium; TMA = Trimethylammonium; BuPy = 1-Butylpyridinium; m-TBT = m-tert-Butyltoluol; p-TBT = p-tert-Butyltoluol; o-TBT = o-tert-Butyltoluol; DTBT = Di-tert-butyltoluol; PhMe = Toluol)

### Beispiel 1

In einem 7 I-Doppelmantelreaktor wurden 3500 g Toluol vorgelegt und auf 50°C erwärmt und anschließend wurden 175 g (1 mol) 1,3-Butylmethylimidazoliumchlorid zugegeben. 267 g (2 mol) Aluminiumchlorid wurden portionsweise innerhalb von 15 min zugegeben. Anschließend wurde 1 h bei 50°C und weitere 1,5 h bei 25°C nachgerührt. Nach Phasentrennung wurden 875 g einer schwarz-grüne Katalysatorphase bestehend aus BMIM [Al₂Cl₇] als 50,5%ige Lösung in Toluol und 3281 g einer grünlichen Toluolphase erhalten.

### Beispiel 2

In einem 7 I-Doppelmantelreaktor wurden 2500 g Toluol vorgelegt und 267 g (2 mol) Aluminiumchlorid wurden zugegeben. Anschließend wurden (1 mol) 138 g Triethylammoniumchlorid innerhalb von 10 min zugegeben. Es wurde 2 h bei 25°C und 2 h bei 45°C nachgerührt. Nach Phasentrennung wurden 895 g einer schwarz-grüne Katalysatorphase bestehend aus Et₃NH [Al₂Cl₇] als 45,3%ige Lösung in Toluol und 2008 g einer grünlichen Toluolphase erhalten.

### Bespiel 3

In einem 7 I-Doppelmantelreaktor wurden 1807 g Toluol vorgelegt und auf 50°C erwärmt und 147 g (1 mol) Ethylmethylimidazoliumchlorid wurden zugegeben. 264 g (1,98 mol) Aluminiumchlorid wurden portionsweise innerhalb von 15 min zugegeben. Es wurde 1h bei 50°C und weitere 1,5 h bei 25°C nachgerührt. Nach Phasentrennung wur-den 864 g einer schwarz-grüne Katalysatorphase bestehend aus EMIM [Al₂Cl₇] als 47,6%ige Lösung in Toluol und 1333 g einer grünlichen Toluolphase erhalten

### Beispiel 4

In einem 7 I-Doppelmantelreaktor wurden 437 g (4,7 mol) Toluol und 4,6 kg (31,3 mol) 4-tert-Butyltoluol vorgelegt. Es wurde Et₃NH Al₂Cl₇ (45%ig in Toluol; 3,2mol% bez. auf 4-tert-Butyltoluol) zugegeben und 1 h bei 20°C gerührt. Nach Ende der Reaktion wur-den die Phasen getrennt. Als untere Phase wurden 631 g des Katalysators Et₃NH Al₂Cl₇ (64%ig in Toluol/3-tert-Butyltoluol/4-tert-Butyltoluol/3,5-Di-tert-butyltoluol) erhalten. Es wurden 5265 g des Produktgemisches (obere Phase) erhalten.
Tabelle 1 zeigt die Zusammensetzung der oberen Phase.

### Beispiele 5 bis 10

Die Reaktion wurde analog Beispiel 4 durchgeführt. Der in Beispiel 4 abgetrennte Katalysator (Et₃NH Al₂Cl₇; 64%ig in Toluol/3-tert-Butyltoluol/4-tert-Butyltoluol/3,5-Di-tert-butyltoluol) wurde wieder eingesetzt. Es wurden 824 g Toluol und 4,1 kg 4-tert-Butyltoluol eingesetzt. Die Reaktion wurde 6mal analog durchgeführt. Tabelle 1 zeigt die Zusammensetzung der oberen Phasen.

**Tabelle 1**

| | Toluol [%] | m-TBT [%] | p-TBT [%] | o-TBT [%] | DTBT [%] |
|---|---|---|---|---|---|
| Beispiel 4 | 20,98 | 43,07 | 21,31 | 0,08 | 11,81 |
| Beispiel 5 | 22,18 | 43,99 | 21,70 | 0,08 | 11,67 |
| Beispiel 6 | 21,53 | 43,18 | 21,25 | 0,08 | 11,50 |
| Beispiel 7 | 21,47 | 42,86 | 21,09 | 0,07 | 11,41 |
| Beispiel 8 | 21,37 | 43,38 | 21,36 | 0,08 | 11,54 |
| Beispiel 9 | 21,47 | 43,07 | 21,55 | 0,08 | 11,65 |
| Beispiel 10 | 21,76 | 43,65 | 21,60 | 0,08 | 11,64 |

### Beispiele 11 bis 35

Die Reaktion wurde analog zu Beispiel 4 durchgeführt. Variiert wurden die eingesetzten Katalysatoren, die Katalysatormengen, Reaktionszeiten, Temperaturen und die Einsatzmengen an Toluol. Tabelle 2 zeigt die Versuchsergebnisse, wobei die Abkürzung eq = Äquivalente bedeutet und FI% Flächenprozente der gaschromatographischen Auswertung sind.

**Tabelle 2**

| Bsp. | Katalysator Stoff mol% | | PhMe [eq] | Zeit min | Temp °C | Toluol FI% | m-TBT FI% | p-TBT FI% | DTBT FI% |
|---|---|---|---|---|---|---|---|---|---|
| 11 | 1 | 5,7 | 1,4 | 30 | 25 | 59,44 | 26,12 | 12,74 | 1,61 |
| 12 | 1 | 13,2 | 1,4 | 30 | 25 | 54,58 | 29,00 | 14,26 | 2,09 |
| 13 | 1 | 13,2 | 1,4 | 140 | 25 | 59,39 | 25,77 | 12,58 | 1,66 |
| 14 | 1 | 1,9 | 1,4 | 30 | 2 | 59,30 | 26,43 | 12,65 | 1,52 |
| 15 | 1 | 2,4 | 2,9 | 30 | 2 | 74,06 | 17,22 | 8,20 | 0,50 |
| 16 | 2 | 2,3 | 1,4 | 30 | 25 | 58,44 | 1,28 | 40,09 | - |
| 17 | 3 | 1,9 | 1,4 | 30 | 25 | 58,51 | 1,28 | 40,07 | - |
| 18 | 4 | 2,1 | 1,4 | 30 | 25 | 58,00 | 1,29 | 40,53 | - |
| 19 | 5 | 2,7 | 1,4 | 30 | 25 | 58,06 | 1,29 | 40,49 | - |
| 20 | 6 | 2,5 | 1,4 | 30 | 25 | 58,70 | 26,48 | 12,93 | 1,59 |
| 21 | 7 | 2,2 | 1,4 | 30 | 25 | 58,95 | 26,40 | 12,87 | 1,57 |
| 22 | 8 | 2,7 | 1,4 | 30 | 25 | 58,69 | 26,10 | 12,63 | 1,49 |
| 23 | 9 | 2,5 | 1,4 | 30 | 25 | 58,05 | 26,48 | 12,93 | 1,65 |
| 24 | 10 | 2,0 | 1,4 | 30 | 25 | 57,13 | 1,32 | 41,35 | - |
| 25 | 7 | 2,7 | 1,4 | 30 | 25 | 56,52 | 3,00 | 39,70 | 0,09 |
| 26 | 11 | 3,1 | 1,4 | 30 | 25 | 57,04 | 1,32 | 41,29 | - |
| 27 | 12 | 2,2 | 1,4 | 30 | 25 | 59,64 | 25,19 | 12,33 | 1,48 |
| 28 | 13 | 2,1 | 1,4 | 30 | 25 | 57,52 | 1,31 | 40,96 | - |
| 29 | 14 | 2,1 | 1,4 | 30 | 25 | 57,74 | 1,30 | 40,78 | - |
| 30 | 15 | 2,5 | 1,4 | 60 | 50 | 58,45 | 1,27 | 40,06 | - |
| 31 | 7 | 2,0 | 1,4 | 120 | 25 | 59,52 | 25,42 | 13,45 | 1,57 |
| 32 | 7 | 2,0 | 0,2 | 30 | 25 | 22,17 | 43,85 | 21,63 | 11,80 |
| 33 | 7 | 1,0 | 0,2 | 12 | 25 | 22,22 | 43,67 | 21,92 | 11,93 |
| 34 | 7 | 0,4 | 0,2 | 30 | 25 | 22,15 | 43,69 | 21,77 | 11,87 |
| 35 | 7 | 0,1 | 0,2 | 60 | 25 | 16,97 | 5,69 | 75,06 | 1,07 |

BMIM Al₂Cl₇ = 1; HMIM Cl = 2; BMIM Cl = 3; EMIM Cl = 4 EMIM OAc = 5; EMIM Al₂Cl₇ = 6; TEA Al₂Cl₇ = 7; TMA Al₂Cl₇ = 8; BuPy Al₂Cl₇ = 9; BMIM InCl₄ = 10; EMIM InCl₄ = 11; HMIM Al₂Cl₇ = 12; HMIM AlCl₄ = 13; HMIM InCl₄ = 14; BMIM AlCl₄ = 15.

### Beispiel 36

Es wurden 4,2 g Toluol und 3,0 g 4-tert-Butyltoluol vorgelegt und EMIM Al₂Cl₇ (45%ig in Toluol; 3mol% bez. auf 4-tert-Butyltoluol) zugegeben und 3 min bei 20°C gerührt. Die Phasen wurden getrennt und aus der oberen Wertproduktphase eine Probe genommen und analysiert. Die Probe hatte die folgende Zusammensetzung: Toluol 57%; 3-tert-Butyltoluol 27%; 4-tert-Butyltoluol 13%; 3,5-Di-tert-butyltoluol 1,7%.

## Patentansprüche

1. Verfahren zur Herstellung m-substituierter Alkyltoluole der Formel (I) 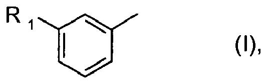 worin R₁ C₁-C₅-Alkyl bedeutet, **dadurch gekennzeichnet, dass** man ein p-substituiertes Alkyltoluol der Formel (II) 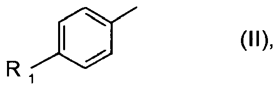 worin R₁ die unter Formel (I) angegebene Bedeutung hat, in Gegenwart von ionischen Flüssigkeiten zu einem m-substituierten Alkyltoluol der Formel (I) isomerisiert.

2. Verfahren gemäß Anspruch 1, **dadurch gekennzeichnet, dass** man ionische Flüssigkeiten verwendet, enthaltend eine Mischung der Komponenten (a) und (b), worin
(a) eine erste Komponente der Formel MX₃ oder MX₂ ist, worin M Metall und X Halogen bedeuten und,
(b) eine zweite Komponente ausgewählt ist aus der Gruppe Alkylammoniumhalogenid, Imidazoliumhalogenid, Pyridiniumhalogenid, Phosphoniumhalogenid und Mischungen dieser Verbindungen.

3. Verfahren gemäß Anspruch 2, **dadurch gekennzeichnet, dass** man ionische Flüssigkeiten verwendet, bestehend aus den Komponenten (a) und (b).

4. Verfahren gemäß einem der Ansprüche 2 und 3, **dadurch gekennzeichnet, dass** das Verhältnis der Komponenten (a) / (b) größer 1 / 1 ist.

5. Verfahren gemäß einem der Ansprüche 2 bis 4, **dadurch gekennzeichnet, dass** man ionische Flüssigkeiten verwendet, worin die Komponente (a) AlCl₃, AlBr₃, ZnCl₂, FeBr₃ und FeCl₃ ist.

6. Verfahren gemäß einem der Ansprüche 2 bis 4, **dadurch gekennzeichnet, dass** man ionische Flüssigkeiten verwendet, worin die Komponente (b) Imidazoliumhalogenid oder Alkylammoniumhalogenid ist.

7. Verfahren gemäß Anspruch 6, **dadurch gekennzeichnet, dass** man als Komponente (b) 1-Ethyl-3-methylimidazoliumchlorid, 1-Butyl-3-methylimidazoliumchlorid, 1-Hexyl-3-methylimidazoliumchlorid, 1-Methylimidazoliumchlorid, Triethylammoniumchlorid, Trimethylammoniumchlorid, Tetramethylammoniumchlorid, Tetraethylammoniumchlorid, 1-Butylpyridiniumchlorid oder 1-Methylpyridiniumchlorid verwendet.

8. Verfahren gemäß einem der Ansprüche 2 bis 7, **dadurch gekennzeichnet, dass** das Verhältnis der Komponenten (a) / (b) grösser 1.5/1 und kleiner 2.1 /1 ist.

9. Verfahren gemäß einem der Ansprüche 1 bis 8, **dadurch gekennzeichnet, dass** die Isomerisierung bei einer Temperatur zwischen 0°C und 100°C erfolgt.

10. Verfahren gemäß einem der Ansprüche 1 bis 9, **dadurch gekennzeichnet, dass** die Isomerisierung in einem Lösungsmittel
durchgeführt wird.

11. Verfahren gemäß Anspruch 10, **dadurch gekennzeichnet, dass** Toluol, Xylole, Dichlormethan, Chlorbenzol, Hexan, Heptan, oder Mischungen dieser Lösungsmittel verwendet werden.

12. Verfahren gemäß einem der Ansprüche 1 bis 11 **dadurch gekennzeichnet, dass** man ein p-substituiertes Alkyltoluol der Formel (II) 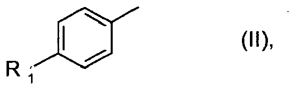 einsetzt, worin R₁ Ethyl, Isopropyl oder tert-Butyl bedeutet.

13. Verfahren gemäß Anspruch 12, **dadurch gekennzeichnet, dass** man als Verbindung der Formel (II)4-tert-Butyltoluol auswählt.

14. Verfahren zur Herstellung von Duftstoffen und Aromachemikalien der allgemeinen Formel (V) 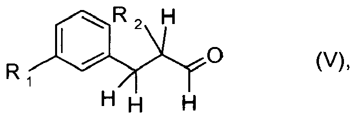 worin R₁ C₁-C₅-Alkyl, bevorzugt C₁-C₄-Alkyl, besonders bevorzugt Ethyl, Isopropyl oder tert-Butyl, und R₂ Wasserstoff oder C₁-C₅-Alkyl, insbesondere Wasserstoff oder Methyl, bedeutet, umfassend die Stufen
a) Isomerisierung eines p-substituierten Alkyltoluols der Formel (II) 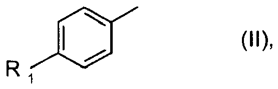 worin R₁ die in Anspruch 1 angegebene Bedeutung hat, in Gegenwart von ionischen Flüssigkeiten zu einem m-substituierten Alkyltoluol der Formel (I) 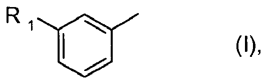 worin R₁ die in Anspruch 1 angegebenen Bedeutungen hat,
b)Oxidation des m-alkylsubstituierten Toluols der Formel (I) zu dem m-alkylsubstituierten Benzaldehyd der Formel (III) 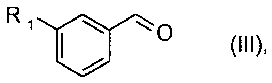 worin R₁ die unter Formel (I) angegebene Bedeutung hat,
c) Aldolkondensation des Benzaldehyds der Formel (III) mit einem Aldehyd der Formel (IIIa) 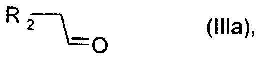 worin R₂ Wasserstoff oder C₁-C₅-Alkyl bedeutet, zu dem Zimtaldehyd der Formel (IV) 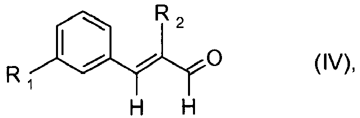 worin R₁ die unter Formel (III) angegebene Bedeutung hat und R₂ die unter Formel (IIIa) angegebene Bedeutung hat, und
d) Hydrierung des Zimtaldehyds der Formel (IV) zu dem alkylsubstituierten Phenylpropanal der Formel (V) 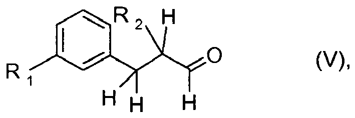 worin R₁ und R₂ die unter Formel (IV) angegebenen Bedeutungen haben.

## Claims

1. A process for the preparation of m-substituted alkyltoluenes of the formula (I) 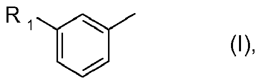 in which R₁ is C₁-C₅-alkyl, wherein a p-substituted alkyltoluene of the formula (II) 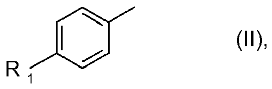 in which R₁ has the meaning given under formula (I), is isomerized in the presence of ionic liquids to give an m-substituted alkyltoluene of the formula (I).

2. The process according to claim 1, wherein ionic liquids are used comprising a mixture of the components (a) and (b), in which
(a) is a first component of the formula MX₃ or MX₂, in which M is metal and X is halogen, and
(b) is a second component selected from the group consisting of alkylammonium halide, imidazolium halide, pyridinium halide, phosphonium halide and mixtures of these compounds.

3. The process according to claim 2, wherein ionic liquids are used consisting of the components (a) and (b).

4. The process according to any one of claims 2 and 3, wherein the ratio of components (a)/(b) is greater than 1/1.

5. The process according to any one of claims 2 to 4, wherein ionic liquids are used in which the component (a) is AlCl₃, AlBr₃, ZnCl₂, FeBr₃ and FeCl₃.

6. The process according to any one of claims 2 to 4, wherein ionic liquids are used in which the component (b) is imidazolium halide or alkylammonium halide.

7. The process according to claim 6, wherein, as component (b), 1-ethyl-3-methylimidazolium chloride, 1-butyl-3-methylimidazolium chloride, 1-hexyl-3-methylimidazolium chloride, 1-methylimidazolium chloride, triethylammonium chloride, trimethylammonium chloride, tetramethylammonium chloride, tetraethylammonium chloride, 1-butylpyridinium chloride or 1-methylpyridinium chloride is used.

8. The process according to any one of claims 2 to 7, wherein the ratio of components (a)/(b) is greater than 1.5/1 and less than 2.1/1.

9. The process according to any one of claims 1 to 8, wherein the isomerization takes place at a temperature between 0°C and 100°C.

10. The process according to any one of claims 1 to 9, wherein the isomerization is carried out in a solvent.

11. The process according to claim 10, wherein toluene, xylenes, dichloromethane, chlorobenzene, hexane or heptane, or mixtures of these solvents are used.

12. The process according to any one of claims 1 to 11, wherein a p-substituted alkyltoluene of the formula (II) 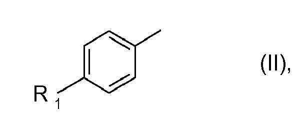 is used, in which R₁ is C₁-C₄-alkyl, preferably ethyl, isopropyl or tert-butyl.

13. The process according to claim 12, wherein 4-tert-butyltoluene is selected as compound of the formula (II).

14. A process for the preparation of fragrances and aroma chemicals of the general formula (V) 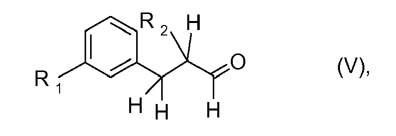 in which R₁ is C₁-C₅-alkyl, preferably C₁-C₄-alkyl, particularly preferably ethyl, isopropyl or tert-butyl, and R₂ is hydrogen or C₁-C₅-alkyl, in particular hydrogen or methyl, comprising the stages
a) isomerization of a p-substituted alkyltoluene of the formula (II) 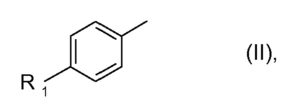 in which R₁ has the meaning given in claim 1, in the presence of ionic liquids to given an m-substituted alkyltoluene of the formula (I) 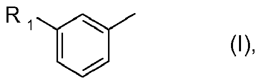 in which R₁ has the meanings given in claim 1,
b) oxidation of the m-alkyl-substituted toluene of the formula (I) to give the m-alkyl-substituted benzaldehyde of the formula (III) 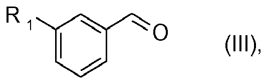 in which R₁ has the meaning given under formula (I),
c) aldol condensation of the benzaldehyde of the formula (III) with an aldehyde of the formula (IIIa) 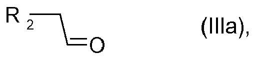 in which R₂ is hydrogen or C₁-C₅-alkyl, to give the cinnamaldehyde of the formula (IV) 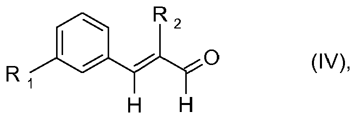 in which R₁ has the meaning given under formula (III) and R₂ has the meaning given under formula (IIIa), and
b) hydrogenation of the cinnamaldehyde of the formula (IV) to give the alkyl-substituted phenylpropanal of the formula (V) 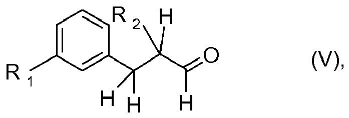 in which R₁ and R₂ have the meanings given under formula (IV).

## Revendications

1. Procédé pour la préparation d'alkyltoluènes m-substitués de formule (I) 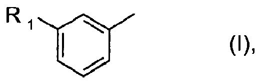 dans laquelle R₁ signifie C₁-C₅-alkyle, **caractérisé en ce qu'**on isomérise un alkyltoluène p-substitué de formule (II) 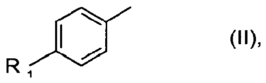 dans laquelle R₁ présente la signification indiquée pour la formule (I) en présence de liquides ioniques en un alkyltoluène m-substitué de formule (I).

2. Procédé selon la revendication 1, **caractérisé en ce qu'**on utilise des liquides ioniques contenant un mélange des composants (a) et (b), dans lequel
(a) est un premier composant de formule MX₃ ou MX₂, dans laquelle M signifie un métal et X signifie un halogène et
(b) est un deuxième composant choisi dans le groupe formé par un halogénure d'alkylammonium, un halogénure d'imidazolium, un halogénure de pyridinium, un halogénure de phosphonium et des mélanges de ces composés.

3. Procédé selon la revendication 2, **caractérisé en ce qu'**on utilise des liquides ioniques, constitués par les composants (a) et (b).

4. Procédé selon l'une quelconque des revendications 2 et 3, **caractérisé en ce que** le rapport des composants (a)/(b) est supérieur à 1/1.

5. Procédé selon l'une quelconque des revendications 2 à 4, **caractérisé en ce qu'**on utilise des liquides ioniques dans lesquels le composant (a) est AlCl₃, AlBr₃, ZnCl₂, FeBr₃ et FeCl₃.

6. Procédé selon l'une quelconque des revendications 2 à 4, **caractérisé en ce qu'**on utilise des liquides ioniques dans lesquels le composant (b) est un halogénure d'imidazolium ou d'alkylammonium.

7. Procédé selon la revendication 6, **caractérisé en ce qu'**on utilise comme composant (b) du chlorure de 1-éthyl-3-méthylimidazolium, du chlorure de 1-butyl-3-méthylimidazolium, du chlorure de 1-hexyl-3-méthylimidazolium, du chlorure de 1-méthylimidazolium, du chlorure de triéthylammonium, du chlorure de triméthylammonium, du chlorure de tétraméthylammonium, du chlorure de tétraéthylammonium, du chlorure de 1-butylpyridinium ou du chlorure de 1-méthylpyridinium.

8. Procédé selon l'une quelconque des revendications 2 à 7, **caractérisé en ce que** le rapport des composants (a)/(b) est supérieur à 1,5/1 et inférieur à 2,1/1.

9. Procédé selon l'une quelconque des revendications 1 à 8, **caractérisé en ce que** l'isomérisation a lieu à une température entre 0°C et 100°C.

10. Procédé selon l'une quelconque des revendications 1 à 9, **caractérisé en ce que** l'isomérisation est réalisée dans un solvant.

11. Procédé selon la revendication 10, **caractérisé en ce qu'**on utilise du toluène, des xylènes, du dichlorométhane, du chlorobenzène, de l'hexane, de l'heptane ou des mélanges de ces solvants.

12. Procédé selon l'une quelconque des revendications 1 à 11, **caractérisé en ce qu'**on utilise un alkyltoluène p-substitué de formule (II) 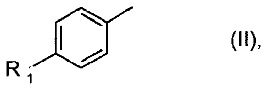 dans laquelle R₁ signifie éthyle, isopropyle ou tert-butyle.

13. Procédé selon la revendication 12, **caractérisé en ce qu'**on choisit comme composé de formule (II) du 4-tert-butyltoluène.

14. Procédé pour la préparation de parfums et de produits chimiques parfumés de formule générale (V) 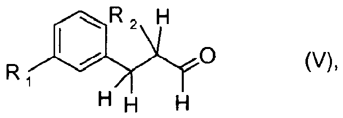 dans laquelle R₁ signifie C₁-C₅-alkyle, de préférence C₁-C₄-alkyle, de manière particulièrement préférée éthyle, isopropyle ou tert-butyle, et R₂ signifie hydrogène ou C₁-C₅-alkyle, en particulier hydrogène ou méthyle, comprenant les étapes de
a) isomérisation d'un alkyltoluène p-substitué de formule (II) 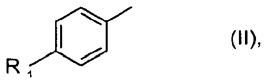 dans laquelle R₁ présente la signification indiquée dans la revendication 1, en présence de liquides ioniques en un alkyltoluène m-substitué de formule (I) 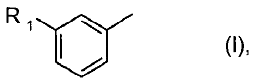 dans laquelle R₁ a les significations indiquées dans la revendication 1,
b) oxydation du toluène m-alkylsubstitué de formule (I) en benzaldéhyde m-alkylsubstitué de formule (III) 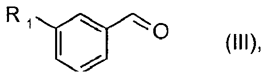 dans laquelle R₁ a la signification indiquée pour la formule (I),
c) condensation aldol du benzaldéhyde de formule (III) avec un aldéhyde de formule (IIIa) 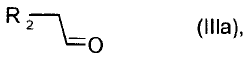 dans laquelle R₂ signifie hydrogène ou C₁-C₅-alkyle, en aldéhyde cinnamique de formule (IV) 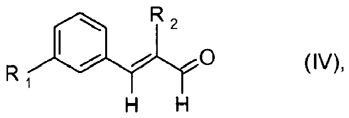 dans laquelle R₁ a la signification indiquée pour la formule (III) et R₂ a la signification indiquée pour la formule (IIIa), et
d) hydrogénation de l'aldéhyde cinnamique de formule (IV) en phénylpropanal alkylsubstitué de formule (V) 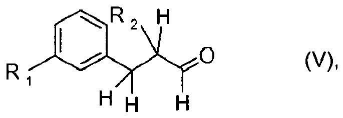 dans laquelle R₁ et R₂ présentent les significations indiquées pour la formule (IV).
